# EUROPÄISCHE PATENTANMELDUNG

(11) **EP 3 950 668 A1**
(43) Veröffentlichungstag der Anmeldung: **09.02.2022**
(21) Anmeldenummer: 21191744.8
(22) Anmeldetag: 11.10.2011
(51) Int. Cl.: C07C 263/10, B01J 19/18

(54) **VERFAHREN ZUR HERSTELLUNG VON ISOCYANATEN**

(30) Priorität: 14.10.2010 EP 10187631
(62) Teilanmeldung aus: 11767258.4
(71) Anmelder: BASF SE, 67056 Ludwigshafen am Rhein (DE)
(72) Erfinder: Mattke, Torsten, 67056 Ludwigshafen (DE); Olbert, Gerhard, 67056 Ludwigshafen (DE)
(74) Vertreter: BASF IP Association

(57) **Zusammenfassung**

Verfahren zur Herstellung von Isocyanaten durch Umsetzung der korrespondierenden Amine mit Phosgen umfassend die Schritte
(a) Bereitstellen mindestens eines Amin enthaltenden Eduktstroms und mindestens eines Phosgen enthaltenden Eduktstroms,
(b) Mischen der Eduktströme zu mindestens einem Reaktionsgemisch in einer Mischzone,
(c) Umsetzen des mindestens einen Reaktionsgemischs in einer Reaktionszone, und
(d) Aufarbeiten des mindestens einen aus (c) erhaltenen Produktgemischs,
wobei
(i) die Mischzone aus mindestens zwei parallel geschalteten und getrennt voneinander regelbaren Strängen jeweils umfassend mindestens eine Mischeinheit aufgebaut ist,
wobei getrennt voneinander regelbar bedeutet, dass die einzelnen, parallel geschalteten Stränge voneinander separat absperrbar sind und jeweils unabhängig voneinander betrieben werden können.

## Beschreibung

Die vorliegende Patentanmeldung beansprucht die Priorität der anhängigen vorläufigen US-Patentanmeldung 61/393,006 mit dem Anmeldetag 14. Oktober 2010, die durch Bezugnahme in ihrer Gesamtheit in die vorliegende Patentanmeldung einbezogen wird.

Die Erfindung betrifft ein Verfahren zur Herstellung von Isocyanaten durch Umsetzung der korrespondierenden Amine mit Phosgen, bei dem Phosgen und Amin zunächst in einer Mischzone gemischt und in einer Reaktionszone zu Isocyanat umgesetzt werden. Dabei sind die Mischzone und/oder die Reaktionszone aus mindestens zwei parallel geschalteten und getrennt voneinander regelbaren Strängen jeweils umfassend mindestens eine Mischeinheit bzw. und/oder mindestens eine Reaktionseinheit aufgebaut.

Die Herstellung von Isocyanaten durch Phosgenierung der korrespondierenden Amine kann prinzipiell durch eine Flüssigphasen- oder eine Gasphasen-Phosgenierung erfolgen. Im Unterschied zur Gasphasen-Phosgenierung wird die Reaktion bei der Flüssigphasen-Phosgenierung bei niedrigen Temperaturen durchgeführt, weiterhin ist eine Verdampfung der Edukte nicht erforderlich.

Bei der Flüssigphasenphosgenierung wird ein aminhaltiger Eduktstrom in der Flüssigphase zugeführt. Dieser wird mit einem phosgenhaltigen Eduktstrom vermischt. Hierbei kann das Phosgen in einem inerten Lösungsmittel gelöst sein. Anschließend wird der phosgenhaltige Eduktstrom in einer Mischeinrichtung eingespritzt, in der dieser mit dem aminhaltigen Eduktstrom vermischt. Das Amin und das Phosgen setzen sich unter Freisetzung von HCl zu den entsprechenden Isocyanaten um.

Eine schnelle Vermischung des Amins mit dem Phosgen ist notwendig, da das entstehende Isocyanat bei einer zu niedrigen Phosgenkonzentration mit dem überschüssigen Amin zu Harnstoff bzw. anderen störenden, hochviskosen und festen Nebenprodukten reagiert. Aus diesem Grund sind eine schnelle Vermischung und eine kurze Verweilzeit in der Reaktionskammer erforderlich.

Ein Verfahren zur Flüssigphasen-Phosgenierung von Aminen zur Herstellung von Isocyanaten ist beispielsweise in der WO 2010/015667 A1 beschrieben.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von HCl zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Aufgrund des geringen Dampfdrucks, insbesondere der Diamine, erfolgt die Verdampfung bei erhöhter Temperatur. Dadurch werden jedoch auch Zersetzungsreaktionen der Amine bzw. Diamine, beispielsweise Desaminierungen, Demethylierungen und Dimerisierungen hervorgerufen, die sich negativ auf die Selektivität des Gesamtprozesses auswirken.

Zudem setzen bei der Kontaktierung der beiden Eduktströme aufgrund der hohen Temperaturen schnell reaktive Umsetzungen ein. Neben der Phosgenierung des Amins zum Isocyanat können unerwünschte Neben- und Folgereaktionen stattfinden. So kann zum Beispiel bereits gebildetes Isocyanat mit noch nicht abreagiertem Amin einen Harnstoff bilden. Des Weiteren können auch Carbodiimide und Cyanurate entstehen. Dies wirkt sich zum einen auf die Selektivität des Prozesses aus, zum anderen können gebildete feste Nebenprodukte zu Verstopfungen führen und sich so negativ auf die Laufzeit der Anlage auswirken. Daher wird im Allgemeinen angestrebt, die Eduktströme möglichst schnell zu vermischen, um Mischverhältnisse, die die Bildung von Nebenkomponenten beschleunigen, soweit wie möglich zu vermeiden.

Ein Verfahren für die Herstellung von (Poly)isocyanaten in der Gasphase mit optimierter Vermischung der Reaktanden ist beispielsweise in der EP 1 319 655 A2 beschrieben.

Somit sind sowohl bei der Gasphasen-Phosgenierung als auch bei der Flüssigphasen-Phosgenierung das Mischen der Edukte sowie die Verweilzeit des Reaktionsgemischs in den entsprechenden Reaktionsräumen kritische Parameter. Die Anlagen zur Herstellung von Isocyanaten durch Phosgenierung von Aminen müssen daher an die speziellen Anforderungen hinsichtlich schneller Durchmischung der Eduktströme und enge Verweilzeitfenster angepasst sein. Anlagen zur Phosgenierung von Aminen werden dabei im Wesentlichen an die maximale Mengenströme bzw. an die jeweilige Nennlast ausgelegt. Dies bedeutet, sowohl Mischorgane, wie Düsen, als auch die Reaktionsräume, beispielsweise Verweilzeitreaktoren, arbeiten bei der Nennlast im optimalen Bereich mit optimierter Ausbeute, Reinheit der Produkte etc. Ist die Anlage jedoch nicht voll ausgelastet, d.h. sie wird nur bei einem Teil der Nennlast betrieben, ändern sich beispielsweise die Verweilzeiten und die Anlage arbeitet nicht mehr im optimalen Bereich. Dies ist beispielsweise bei An- und Abfahrvorgängen, Teilauslastung der Anlage oder Störungen in der Anlage der Fall. In diesen Fällen verminderter Last arbeiten sowohl die Mischorgane als auch die Verweilzeitreaktoren nicht im optimalen Bereich. Die Folge sind Ausbeuteverluste, Foulingprobleme und/oder Qualitätseinbußen.

Aufgabe der vorliegenden Erfindung war daher, ein Verfahren zur Herstellung von Isocyanaten durch Umsetzung der korrespondierenden Amine mit Phosgen bereit zu stellen, das auch bei unterschiedlichen Auslastungen ohne die vorstehend beschriebenen Probleme durchgeführt werden kann, insbesondere soll auch bei Fahren der Anlage im Teillastbereich das Mischen und/oder die Reaktion in dem jeweils optimierten Verweilzeitfenster erfolgen.

Diese Aufgabe wird erfindungsgemäß gelöst durch das folgende Verfahren zur Herstellung von Isocyanaten durch Umsetzung der korrespondierenden Amine mit Phosgen umfassend die Schritte
(a) Bereitstellen mindestens eines Amin enthaltenden Eduktstroms und mindestens eines Phosgen enthaltenden Eduktstroms,
(b) Mischen der Eduktströme zu mindestens einem Reaktionsgemischs in einer Mischzone,
(c) Umsetzen des mindestens einen Reaktionsgemischs in einer Reaktionszone, und
(d) Aufarbeiten des aus (c) erhaltenen Produktgemischs,
wobei
(i) die Mischzone aus mindestens zwei parallel geschalteten und getrennt voneinander regelbaren Strängen jeweils umfassend mindestens eine Mischeinheit aufgebaut ist, oder
(ii) die Reaktionszone aus mindestens zwei parallel geschalteten und getrennt voneinander regelbaren Strängen jeweils umfassend mindestens eine Reaktionseinheit aufgebaut ist, oder
(iii) die Mischzone und die Reaktionszone aus mindestens zwei parallel geschalteten und getrennt voneinander regelbaren Strängen jeweils umfassend mindestens eine Mischeinheit und mindestens eine Reaktionseinheit aufgebaut sind.

"Getrennt voneinander regelbar" bedeutet im Rahmen der Erfindung, dass die einzelnen, parallel geschalteten Stränge voneinander separat absperrbar sind und jeweils unabhängig voneinander betrieben werden können.

Als "Einheit" (Mischeinheit, Reaktionseinheit bzw. Quencheinheit) wird im Rahmen der Erfindung jeweils eine Vorrichtung verstanden, in der der jeweilige Verfahrensschritt (Mischen, Umsetzen bzw. Quenchen) durchgeführt werden kann. Als Reaktionseinheit kann beispielsweise ein Rohrreaktor eingesetzt werden, als Mischeinheit ein dynamischer Mischer mit Rotor/Statorsystem und als Quencheinheit eine für das Quenchen geeignete Vorrichtung.

Je nach Anzahl N der parallelen Stränge werden Mischeinheit(en) bzw. Reaktionseinheit(en) auf eine Kapazität von 1/N der Gesamtkapazität ausgelegt. Bei einer Teillast-Fahrweise wird nur die erforderliche Anzahl an Strängen in Betrieb genommen, wobei diese Stränge jeweils im optimalen Bereich laufen. Dabei können die einzelnen Stränge jeweils die gleiche Kapazität besitzen, d.h. für die gleiche Nennlast ausgelegt sein, die einzelnen Stränge können jedoch auch unterschiedliche Kapazitäten aufweisen, bzw. für unterschiedliche Nennlasten ausgelegt sein. Erfindungsgemäß bevorzugt sind die Mischzone und die Reaktionszone jeweils aus zwei Strängen mit jeweils gleicher Kapazität, drei Stränge mit jeweils gleicher Kapazität oder vier Stränge mit jeweils gleicher Kapazität aufgebaut. Ein Strang kann dabei ein oder mehrere Einheiten der gleichen Sorte, d.h. Mischeinheiten, Reaktionseinheiten, Quencheinheiten umfassen, die hintereinander oder parallel geschaltet sind.

Mit dem erfindungsgemäßen Verfahren können in einer Anlage unterschiedliche Mengen an gewünschtem Produkt hergestellt werden, wobei jedoch stets die optimalen Betriebsparameter für die einzelnen Stränge eingehalten werden können, so dass jeweils eine gute und schnelle Durchmischung der Eduktströme erfolgt und/oder die Verweilzeiten der Eduktströme bzw. des Produktgemischs entsprechend der für die einzelnen Stränge vorteilhaften Werte eingehalten werden. Dies führt zu weniger unerwünschten Nebenprodukten. Gleichzeitig können jedoch die Vorteile einer gemeinsamen Aufarbeitung der Produktströme in einem Strang genutzt werden, beispielsweise durch Einsparung von zusätzlichen Rohren, Destillationsapparaturen, Wäschern etc. Auch treten weniger Foulingprobleme auf. Weiterhin ermöglicht das erfindungsgemäße Verfahren vergleichsweise einfach eine nachträgliche Kapazitätsaufstockung, da ein oder mehrere neue Stränge umfassend eine oder mehrere Mischeinheiten, Reaktionseinheiten und/oder Quencheinheiten bzw. weitere Einheiten zur Aufarbeitung aufgebaut werden können, während die bereits vorhandenen Strangs im Wesentlichen weiterbetrieben werden können.

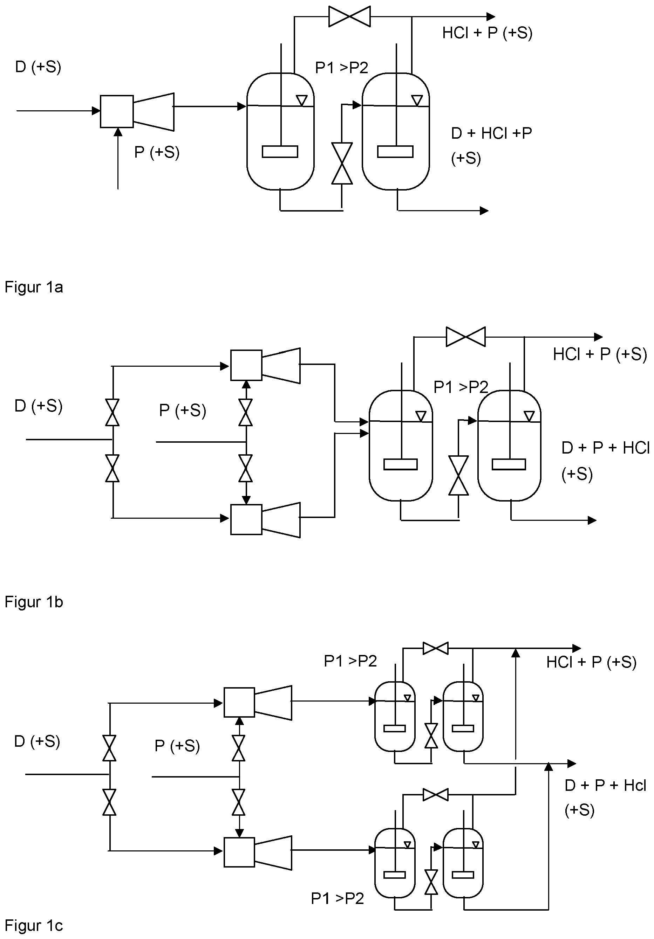

Falls lediglich die Mischzone aus mindestens zwei parallel geschalteten und getrennt voneinander regelbaren Strängen aufgebaut ist (Variante (i)), erfolgt die Reaktion in einer gemeinsamen Reaktionszone aufgebaut aus mindestens einer Reaktionseinheit. Dabei können die aus den mindestens zwei Strängen umfassend jeweils mindestens eine Mischeinheit stammenden Reaktionsgemische vor Eintritt in die Reaktionszone vereinigt werden, es ist jedoch auch möglich, die mindestens zwei Reaktionsgemische getrennt der Reaktionszone zuzuführen und in der Reaktionszone zu vereinigen. Dabei gibt es jeweils so viele Reaktionsgemische wie Stränge, die je nach Auslastung in Betrieb genommen sind. Eine Ausführungsform der Variante (i) des erfindungsgemäßen Verfahrens ist schematisch in Figur 1b dargestellt.

Falls die Reaktionszone, jedoch nicht die Mischzone aus mindestens zwei parallel geschalteten Strängen aufgebaut ist (Variante (ii)), wird das in Schritt (b) erhaltene Reaktionsgemisch je nach Auslastung auf einen oder mehr der mindestens zwei parallel geschalteten Stränge umfassend jeweils mindestens eine Reaktionseinheit, aus denen die Reaktionszone aufgebaut ist, aufgeteilt.

Erfindungsgemäß bevorzugt ist sowohl die Mischzone als auch die Reaktionszone aus mindestens zwei parallel geschalteten und getrennt voneinander regelbaren Strängen aufgebaut, wobei jeder Strang mindestens eine Mischeinheit und mindestens eine Reaktionseinheit umfasst (Variante (iii)). Eine Ausführungsform der Variante (iii) des erfindungsgemäßen Verfahrens ist schematisch in Figur 1c dargestellt.

Zur Herstellung des Isocyanats werden der mindestens eine Phosgen enthaltende Eduktstrom und der mindestens eine Amin enthaltende Eduktstrom zunächst zur Mischzone zugeführt, in der die Vermischung von Amin enthaltenden Edukstrom und Phosgen enthaltenden Eduktstrom zu einem Reaktionsgemisch erfolgt (Schritt (b)). Dabei muss auf eine ausreichend schnelle Vermischung der Reaktanden geachtet werden. Methoden zur Durchführung kurzer Mischzeiten sind im Prinzip bekannt. In den Mischeinheiten können Mischaggregate mit dynamischen oder statischen Mischern eingesetzt werden. Erfindungsgemäß bevorzugt werden in den Mischeinheiten ein oder mehrere statische Mischorgane eingesetzt. Als statische Mischorgane geeignet sind beispielsweise aus der Verbrennungstechnik bekannte Düsen, Glattstrahldüsen oder Venturidüsen, sowie Lavaldüsen. Eine besonders vorteilhafte Ausführung eines statischen Mischorgans ist in WO2010/015667 A1 beschrieben. Als dynamische Mischer können beispielsweise in den Mischeinheiten angeordnete Rotor/Statorsysteme eingesetzt werden. Erfindungsgemäß bevorzugt werden statische Mischorgane, insbesondere Düsen eingesetzt.

Nach dem Mischen der Eduktströme zu mindestens einem Reaktionsgemisch wird das Reaktionsgemisch in einer Reaktionszone umfassend mindestens eine Reaktionseinheit umgesetzt (Schritt (c)). Reaktoren, die als Reaktionseinheiten zur Phosgenierung eines Amins zur Herstellung von Isocyanaten eingesetzt werden können, sind dem Fachmann bekannt. Vorzugsweise umfasst eine Reaktionszone jeweils mindestens einen Verweilzeitreaktor. Vorzugsweise werden als Verweilzeitreaktoren Raktionskolonnen, Rohrreaktoren und/oder Rührkesselkaskaden eingesetzt.

In der Reaktionszone wird das Amin mit dem Phosgen zum korrespondierenden Isocyanat und Chlorwasserstoff umgesetzt. Üblicherweise wird das Phosgen im Überschuss zugegeben, so dass das in der Reaktionszone entstehende Reaktionsgemisch neben dem gebildeten Isocyanat mit dem Chlorwasserstoff auch Phosgen enthält.

Im Anschluss an Schritt (c) erfolgt in Schritt (d) die Aufarbeitung des in Schritt (c) erhaltenen Produktgemischs.

Gemäß einer bevorzugten Ausführungsform der Erfindung wird die Aufarbeitung in Schritt (d) des erfindungsgemäßen Verfahrens in einem gemeinsamen Strang durchgeführt. Dies bedeutet, dass die aus den mindestens zwei parallel geschalteten und jeweils getrennt voneinander regelbaren Strängen aufgebauten Mischzonen und/oder Reaktionszonen stammenden Produktgemische ggf. vereinigt und gemeinsam in einem Strang aufgearbeitet werden. Abtrennung der gewünschten Isocyanate und die Abtrennung der gegebenenfalls im Produktgemisch enthaltenen Lösungsmittel, Inertgase, Edukte und Quenchmedien, gegebenenfalls durchgeführte Wäschen des Produktgemisches und Kondensationen erfolgen gemeinsam.

Wird ein parallel geschalteter Strang umfassend ein oder mehrere Mischeinheiten, Reaktionseinheiten und/oder Quencheinheiten bzw. weitere Einheiten für die Aufarbeitung abgeschaltet, wird der betreffende Strang bzw. die eine oder mehr betreffenden Einheiten nach dem Abschalten üblicherweise gespült, um ggf. vorhandene Eduktreste, Produktreste etc. wiederzugewinnen und/oder den abgeschalteten Strang bzw. die abgeschalteten Einheiten zu reinigen. Umgekehrt können bei Zuschaltung eines zuvor nicht im Betrieb befindlichen parallel geschalteten Strangs umfassend ein oder mehrere Mischeinheiten, Reaktionseinheiten und/oder Quencheinheiten bzw. weiterer Einheiten für die Aufarbeitung des mindestens einen aus Schritt (c) erhaltenen Produktgemischs diese Einheiten vor Zuschalten mit den entsprechenden Edukten/Reaktionsmischungen etc. gespült und/oder befüllt werden. Die nach dem erfindungsgemäßen Verfahren vorhandenen mindestens zwei parallel geschalteten und getrennt voneinander regelbaren Stränge umfassen daher in der Regel auch die entsprechenden, dem Fachmann zum Spülen/Zuführen etc. üblicherweise eingesetzten Vorrichtungen.

Das erfindungsgemäße Verfahren eignet sich sowohl für die Gasphasen-Phosgenierung als auch für die Flüssigphasen-Phosgenierung.

Gemäß einer Ausführungsform der Erfindung erfolgt die Umsetzung von Amin und Phosgen in der Reaktionszone in der Gasphase. Hierzu liegt der Druck in der Reaktionszone üblicherweise im Bereich zwischen 0,3 bis 3 bar absolut, bevorzugt im Bereich von 0,8 bis 3,0 bar absolut. Die Temperatur liegt dabei üblicherweise im Bereich von 250 bis 550°C, bevorzugt im Bereich von 300 bis 500°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck in der Mischzone liegt vorzugsweise in einem Bereich von 0,05 bis 3 bar absolut und die Temperatur in der Mischzone in einem Bereich von 200 bis 400 °C. Die Temperatur in der Mischzone ergibt sich dabei aus der Temperatur des in der Mischzone einströmenden Phosgens und Amins. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur durch Absenkung des Drucks des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C. Es ist jedoch auch möglich, Wasserdampf mit einer Temperatur von mehr als 311°C zur Verdampfung des Amins einzusetzen.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Bevorzugt wird der mindestens eine Amin enthaltende Eduktstrom und der mindestens eine Phosgen enthaltende Eduktstrom jeweils in mindestens einer Verdampfungszone in die gasförmige Phase überführt und gegebenenfalls in mindestens einer Überhitzungszone weiter überhitzt. Die Verdampfungszone und/oder die Überhitzungszone können aus mindestens zwei parallel geschalteten und getrennt voneinander regelbaren Strängen jeweils umfassend mindestens eine Verdampfungseinheit bzw. und/oder mindestens eine Überhitzungseinheit aufgebaut sein. Bevorzugt sind sowohl Verdampfungszone als auch Überhitzungszone jeweils aus einem Strang aufgebaut.

Dabei kann der mindestens eine Amin enthaltende Eduktstrom in einer Verdampfungszone, die aus mindestens zwei parallel geschalteten und getrennt voneinander regelbaren Strängen umfassend jeweils mindestens eine Verdampfungseinheit aufgebaut ist, verdampft werden. Der mindestens eine Amin enthaltende Eduktstrom kann auch in mindestens einer Überhitzungszone, die aus mindestens zwei parallel geschalteten und getrennt voneinander regelbaren Strängen umfassend jeweils mindestens eine Überhitzungseinheit aufgebaut ist, überhitzt werden. Bevorzugt wird der mindestens eine Amin enthaltende Eduktstrom sowohl in mindestens einer Verdampfungszone in die gasförmige Phase überführt und in mindestens einer Überhitzungszone, überhitzt, die jeweils aus mindestens zwei parallel geschalteten und getrennt voneinander regelbaren Strängen jeweils umfassend mindestens eine Verdampfungseinheit und mindestens eine Überhitzungseinheit aufgebaut sind.

Gleiches gilt für den mindestens einen Phosgen enthaltenden Eduktstrom. Bevorzugt werden sowohl der mindestens eine Amin enthaltende Eduktstrom sowie der mindestens eine Phosgen enthaltende Eduktstrom jeweils in mindestens einer Verdampfungszone in die gasförmige Phase überführt und in mindestens einer Überhitzungszone überhitzt. Sowohl die Verdampfungszone als auch die Überhitzungszone können jeweils aus mindestens zwei parallel geschalteten und getrennt voneinander regelbaren Strängen jeweils umfassend mindestens eine Verdampfungseinheit und mindestens eine Überhitzungseinheit aufgebaut sein. Bevorzugt sind sowohl Verdampfungszone als auch Überhitzungszone jeweils aus einem Strang aufgebaut.

Die Umsetzung in der Gasphase kann in Gegenwart mindestens eines Inertmediums durchgeführt werden. Das Inertmedium kann dabei dem Phosgen enthaltenden Eduktstrom und/oder dem Amin enthaltenden Eduktstrom zugegeben werden. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphthalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Im Allgemeinen wird das Inertmedium in einer Menge zugesetzt, so dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten sich diese oder andere Komponenten aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere bei Auftreten der Aminhydrochloride, die sich aus freien Aminogruppen und Chlorwasserstoff bilden, da die resultierenden Aminhydrochloride leicht ausfallen und nur schwer wieder verdampfbar sind.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas vorzugsweise unmittelbar nach der Reaktion in einem Quench abgekühlt. Hierzu wird ein vorzugsweise flüssiges Quenchmedium zugegeben. Durch Verdampfung des Quenchmediums nimmt dieses Wärme auf und führt zu einer schnellen Abkühlung des Reaktionsgases.

Gemäß einer bevorzugten Ausführungsform des Verfahrens wird bei der Gasphasen-Phosgenierung das mindestens eine aus Schritt (c) erhaltene Produktgemisch in mindestens einer Quenchzone abgekühlt. Das Quenchen wird in einer Quenchzone durchgeführt, die aus einer oder mindestens zwei parallel geschalteten und getrennt voneinander regelbaren Strängen umfassend jeweils mindestens eine Quencheinheit aufgebaut ist. Bei der erfindungsgemäßen Durchführung der Phosgenierung in der Gasphase kann somit die Mischzone (Schritt (b)) und/oder die Reaktionszone (Schritt (c)) und/oder das Quenchen (als Teil der Aufarbeitung des aus Schritt (c) erhaltenen mindestens einen Reaktionsgemischs in Schritt (d)) in mindestens zwei parallel geschalteten und getrennt voneinander regelbaren Strängen aufgebaut sein, wobei die Stränge je nach Ausführung mindestens eine Mischeinheit und/oder mindestens eine Reaktionseinheit sowie mindestens eine Quencheinheit umfassen.

Besonders bevorzugt wird das erfindungsgemäße Verfahren so durchgeführt, dass die Mischzone, die Reaktionszone und die Quenchzone aus mindestens zwei parallel geschalteten und getrennt voneinander regelbaren Strängen aufgebaut sind, wobei jeder dieser parallel geschalteten Stränge mindestens eine Mischeinheit, mindestens eine Reaktionseinheit und mindestens eine Quencheinheit umfasst. Beispielsweise kann das Verfahren mit zwei parallel geschalteten und getrennt voneinander regelbaren Strängen durchgeführt werden, wobei jeder der beiden Stränge mindestens eine Mischeinheit, mindestens eine Reaktionseinheit und mindestens eine Quencheinheit umfasst, so dass die Mischzone, die Reaktionszone und die Quenchzone jeweils aus zwei parallel geschalteten und getrennt voneinander regelbaren Strängen aufgebaut sind. Ein Strang kann dabei ein oder mehrere Einheiten der gleichen Sorte, d.h. Mischeinheiten, Reaktionseinheiten, Quencheinheiten) umfassen, die hintereinander oder parallel geschaltet sind.

Eine schnelle Abkühlung wird insbesondere dadurch erzielt, dass das Quenchmedium fein zerstäubt zugegeben wird. Hierdurch weist das Quenchmedium eine große Oberfläche auf und kann schnell die Wärme aufnehmen und damit das Reaktionsgas abkühlen.

Insbesondere bei Verwendung eines Quenchmediums, das bei den Bedingungen im Quenchraum eine Siedetemperatur unterhalb der Kondensationstemperatur des Reaktionsgases aufweist, ist der Druck in den Zuleitungen gegenüber dem Druck im Quenchraum erhöht, um ein Verdampfen des Quenchmediums vor der Zugabe in den Quenchraum zu vermeiden.

Der Druck, mit dem das Quenchmedium zugegeben wird, liegt vorzugsweise im Bereich von 1 bis 20 bar, mehr bevorzugt im Bereich von 1 bis 10 bar und insbesondere im Bereich von 1 bis 8 bar.

Das zur Kühlung eingesetzte Quenchmedium enthält vorzugsweise ein Lösungsmittel, das ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

Bevorzugt enthält das Quenchmedium einen Teil des im Quench abgekühlten Produktstroms, besonders bevorzugt wird als Quenchmedium ein Teil des bereits im Quench abgekühlten Produktstroms eingesetzt. In diesem Fall enthält das Quenchmedium üblicherweise kein Lösungsmittel sonders lediglich die beim Quenchen auskondensierten Teile des Produktstroms.

Um zu vermeiden, dass sich in Rohrleitungen, Regeleinrichtungen und sonstigen Apparateteilen, insbesondere in den Zerstäuberdüsen des Quenchs Ablagerungen bilden, werden gegebenenfalls im Quenchmedium enthaltene Feststoffpartikel vor Zugabe in den Quench entfernt.

Wenn ein Isocyanat im Quenchmedium enthalten ist, so ist es insbesondere bevorzugt, wenn das bei der Reaktion gebildete Isocyanat zunächst im Quench und gegebenenfalls in nachfolgenden Kühlstufen abgekühlt wird und nach der Abkühlung ein Teilstrom als Quenchmedium eingesetzt wird.

Es ist bevorzugt, wenn das Quenchmedium flüssig zugegeben wird, um eine schnelle Abkühlung des Reaktionsgases im Quench zu erzielen. Die Temperatur des Quenchmediums liegt dabei vorzugsweise im Bereich von 0 bis 250°C, insbesondere im Bereich von 20 bis 220°C. Durch das Einbringen des Quenchmediums in das heiße Reaktionsgas wird das Quenchmedium erwärmt und/oder verdampft. Die für das Erwärmen und die Verdampfung des Quenchmediums notwendige Wärme wird dem Reaktionsgas entzogen und das Reaktionsgas wird auf diese Weise abgekühlt. Die Temperatur, auf die das Reaktionsgas abgekühlt wird, lässt sich zum Beispiel durch die Menge und die Temperatur des zugegebenen Quenchmediums einstellen.

Um die Temperatur, mit der das Quenchmedium in den Quench zugegeben wird, falls erforderlich einzustellen, wird das Quenchmedium vorzugsweise durch einen Wärmetauscher geleitet. Je nach Eintrittstemperatur des Quenchmediums in dem Wärmetauscher kann das Quenchmedium in diesem erwärmt oder abgekühlt werden. Ein Abkühlen ist zum Beispiel dann erforderlich, wenn ein Teil des Produktstromes, der als Quenchmedium eingesetzt wird, direkt im Anschluss an den Quench entnommen wird. Ein Erwärmen kann sich zum Beispiel dann ergeben, wenn der Teil des Produktstromes, der als Quenchmedium eingesetzt wird, am Ende der Aufbereitungsstrecke entnommen wird und eine Temperatur aufweist, die niedriger ist als die gewünschte Temperatur, mit der das Quenchmedium in den Quench zugegeben werden soll. Im Allgemeinen wird es jedoch erforderlich sein, das Quenchmedium vor Zugabe in den Quench abzukühlen.

Wenn das Quenchmedium Lösungsmittel enthält, ist es bevorzugt, dem Quenchmedium vor Zugabe in den Quench das/die Lösungsmittel zuzugeben. Hierdurch können Lösungsmittelverluste im Quenchmedium ausgeglichen werden. Geeignete Lösungsmittel, die im Quenchmedium enthalten sein können, sind zum Beispiel gegebenenfalls mit Halogen substituierte Kohlenwasserstoffe. Vorzugsweise ist das im Quenchmedium enthaltene Lösungsmittel ausgewählt aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Dimethylisophthalat, Tetrahydroforan, Dimethylformamid, Xylol, Chlornaphthalin, Decahydronaphtalin und Toluol.

In einer bevorzugten Ausführungsform schließen sich zur weiteren Behandlung an den Quench weitere Stufen zur Abkühlung des Reaktionsgases an. In den einzelnen Stufen zur Abkühlung erfolgt dabei jeweils eine weitere Abkühlung des Produktstromes bis zum Erreichen der gewünschten Temperatur, mit der der Produktstrom beispielsweise einer nachfolgenden Aufarbeitung zugeführt wird. Als Produktstrom wird im Quench dabei vorzugsweise der gesamte, den Quench verlassende Strom eingesetzt, der sowohl das Quenchmedium, als auch das Reaktionsgemisch enthält.

Die weiteren Stufen zur Abkühlung, die sich an den Quench anschließen können, können zum Beispiel weitere Quenche oder Kondensatoren oder beliebige andere Stufen zur Abkühlung, die dem Fachmann bekannt sind, sein. Bevorzugt ist mindestens eine der sich an den Quench anschließenden Stufen zur Abkühlung des Produktstromes ein Kondensator. Als Kondensator eignet sich dabei jede beliebige, dem Fachmann bekannte Kondensatorbauart. Üblicherweise wird als Kondensator ein Wärmetauscher eingesetzt, der von einem Kühlmedium durchströmt wird. Als Kühlmittel kann zum Beispiel Wasser eingesetzt werden. In diesem Fall kondensiert das Gas zumindest teilweise an den Wandungen des Kondensators aus. Die so entstehende Flüssigkeit läuft ab und wird gesammelt und dem Kondensator entnommen.

Nach dem Kondensieren des Produktstromes wird im Allgemeinen eine Aufbereitung angeschlossen. So ist es zum Beispiel möglich, dass das auskondensierte Gemisch in einem Lösungsmittel gewaschen wird. Als Lösungsmittel können zum Beispiel die gleichen Stoffe eingesetzt werden, die auch als Quenchmedium eingesetzt werden können.

Weiterhin ist es zum Beispiel möglich, das den Quench und die sich gegebenenfalls daran anschließenden Stufen zur Abkühlung verlassende Reaktionsgas vorzugsweise bei Temperaturen von mehr als 130°C mit einem Lösungsmittel zu waschen. Als Lösungsmittel eignen sich zum Beispiel die gleichen Stoffe, die auch als Quenchmedium eingesetzt werden können.

Alternativ zur Abkühlung des Produktstromes ist es auch möglich, dass der Produktstrom nach Austritt aus dem Quench einer Trennstufe zugeführt wird. Eine entsprechende Trennstufe kann sich alternativ jedoch auch zum Beispiel dem Kondensator anschließen. Bevorzugt schließt sich jedoch die Trennstufe direkt an den Quench an. Als Trennstufen eignen sich zum Beispiel Destillationskolonnen oder Wäscher.

Wenn die Trennstufe ein Wäscher ist, so wird der den Quench verlassende Produktstrom vorzugsweise mit einem Lösungsmittel gewaschen. Hierbei wird das Isocyanat selektiv in die Waschlösung überführt. Anschließend werden das verbleibende Gas und die erhaltene Waschlösung bevorzugt mittels Rektifikation in Isocyanat, Lösungsmittel, Phosgen und Chlorwasserstoff aufgetrennt. Als Wäscher eignet sich insbesondere ein Waschturm, in dem aus dem gasförmigen Produktstrom das gebildete Isocyanat durch Kondensation in einem inerten Lösungsmittel abgetrennt wird, während überschüssiges Phosgen, Chlorwasserstoff und gegebenenfalls das Inertmedium den Waschturm gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Als Wäscher eignet sich jeder beliebige, dem Fachmann bekannte Wäscher. So können zum Beispiel Rührbehälter oder andere herkömmliche Apparaturen, beispielsweise Kolonnen oder Mixer-Settler-Apparaturen eingesetzt werden.

Das Waschen und die Aufarbeitung des den Quench verlassenen Gemischs aus Reaktionsgas und Quenchmedium erfolgt dabei im Allgemeinen wie beispielsweise in WO-A 2007/028715 beschrieben.

Wenn die Trennstufe eine Destillationskolonne, auch Rektifikationskolonne genannt, ist, so wird der gasförmige Produktstrom der Rektifikationskolonne zugeführt. Die Rektifikationskolonne wird vorzugsweise so betrieben, dass die Temperatur am Kopf der Rektifikationskolonne niedriger ist als die Siedetemperatur des Produktstromes. Auf diese Weise kondensieren in der Destillationskolonne selektiv einzelne Bestandteile des Produktstromes aus und können der Kolonne am Sumpf, über Kopf und gegebenenfalls über Seitenabzüge entnommen werden.

Wenn zur Aufbereitung des Produktstromes ein Kondensator eingesetzt wird, ist es bevorzugt, das Quenchmedium dem Kondensator zu entnehmen. Bei einer Aufbereitung durch Rektifikation erfolgt vorzugsweise eine Abtrennung des als Quenchmedium eingesetzten Lösungsmittels. Das Lösungsmittel enthält in diesem Fall noch Anteile an Isocyanaten. Das so abgetrennte Gemisch aus Lösungsmittel und Isocyanat wird dann als Quenchmedium eingesetzt.

Wenn ein Teil des Produktstromes als Quenchmedium eingesetzt wird, so ist es möglich, diesen Teil zum Beispiel nach dem Abkühlen aus dem Produktstrom abzuzweigen. Alternativ kann das Quenchmedium auch nach einer sich an den Quench anschließenden Aufarbeitung aus einem beliebigen Strom abgezweigt werden.

Gemäß einer weiteren Ausführungsform der Erfindung erfolgt die Umsetzung in flüssiger Phase. Diese Ausführungsform wird nachstehend genauer beschreiben. Erfindungsgemäß bevorzugt liegt in dem mindestens einen Amin enthaltenden Eduktstrom das Amin als Lösung oder als Suspension vor.

Als Eduktströme für das erfindungsgemäße Verfahren mit Flüssigphasen-Phosgenierung werden üblicherweise einerseits 3 gew.-%ige bis 100 gew.-%ige, vorzugsweise 50 gew.-%ige bis 100 gew.-%ige Phosgenlösungen, und andererseits 5 gew.-%ige bis 95 gew.-%ige Lösungen bzw. Suspensionen von Aminen bzw. deren Salzen in geeigneten Lösungsmitteln eingesetzt.

Geeignete Lösungsmittel zur Herstellung der Phosgen- und Aminlösungen bzw. Suspensionen sind beliebige, unter den Reaktionsbedingungen inerte Lösungsmittel wie beispielsweise Monochlorbenzol, o-Dichlorbenzol, Trichlorbenzol, Toluol, Xylol, Methylenchlorid, Perchlorethylen, Trichlorfluoromethan, Butylacetat, Hexan, Heptan, Octan, Biphenyl, Essigsäureethylester, 1,2-Diacetoxyethan, 2-Butanon, Acetonitril und Sulfan. Beliebige Gemische der beispielhaft genannten Lösungsmittel können selbstverständlich auch eingesetzt werden. Zweckmäßigerweise wird man für die Aminkomponente und das Phosgen das gleiche Lösungsmittel bzw. Lösungsmittelgemisch einsetzen, obwohl dies im Rahmen der Erfindung nicht zwingend erforderlich ist.

Die Zuführung der Edukte wird gemäß einer bevorzugten Ausführungsform der Erfindung so eingestellt und/oder geregelt, dass die Phosgen- und Aminlösungen bzw. Aminsuspensionen in solchen Mengen in die Mischkammer geleitet werden, dass in dieser ein Molverhältnis Phosgen zu primären Aminogruppen von ca. 15:1 bis 1:1, bevorzugt 10:1 bis 2:1 vorliegt.

Gemäß einer bevorzugten Ausführungsform ist die Lösung von Phosgen, also der phosgenhaltige Eduktstrom, frei von Isocyanaten. Dies bedeutet, dass in dem phosgenhaltigen Eduktstrom Isocyanate in einer Menge von kleiner oder gleich 5 Gew.-%, vorzugsweise kleiner 2 Gew.-%, insbesondere kleiner 1 Gew.-% vorhanden sind. Es ist besonders bevorzugt, dass in dem phosgenhaltigen Eduktstrom keine Isocyanate enthalten sind, also diese mit den üblichen Analysemethoden nicht nachgewiesen werden können. Vorteilhafterweise lässt sich hierdurch die Bildung von Reaktionsnebenprodukten wie Harnstoffderivaten, welche sich negativ auf die Selektivität des Prozesses auswirkt und zur Verschmutzung der Anlage bis hin zur Verblockung führen kann, deutlich verringern. Die Bildung von Harnstoffderivaten wird somit verringert, indem dem Verfahren keine oder im Wesentlichen keine Isocyanate, die in Kontakt mit Aminen zur Bildung von Harnstoffderivaten führen können, als Edukte zugeführt werden.

Bei der Durchführung des Verfahrens mit Flüssigphasen-Phosgenierung wird die Temperatur in der Mischzone üblicherweise auf einer Temperatur oberhalb der Zersetzungstemperatur des Carbamoylchlorids des verwendeten Amins gehalten. Für die meisten Amine ist wird das erfindungsgemäße Verfahren bei einer Temperatur von etwa 30 °C bis 300 °C, bevorzugt von etwa 40 °C bis 150 °C, besonders bevorzugt von etwa 50 °C bis 120 °C durchgeführt.

Bei der erfindungsgemäßen Phosgenierung in der Flüssigphase wird mindestens ein Produktgemisch erhalten, das üblicherweise direkt der Aufarbeitung zugeführt wird und dabei teilweise in HCl, Phosgen, Lösungsmittel sowie gebildete Produkte und Nebenprodukte aufgetrennt wird.

Generell können die dem Fachmann zur Herstellung von Isocyanaten bekannten Amine in dem erfindungsgemäßen Verfahren eingesetzt werden. Dabei handelt es sich beispielsweise um Monoamine, Diamine, Triamine und höherwertige Amine. Bevorzugt werden Monoamine und Diamine eingesetzt, besonders bevorzugt Diamine. Entsprechend der eingesetzten Amine ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Amine und Isocyanate können aliphatisch, cycloaliphatisch oder aromatisch sein. Bevorzugt sind die Amine aliphatisch oder cycloaliphatisch, besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylendi(phenylisocyanat) (MDI) sowie dessen höhere Oligomere (Polymehylen(phenalisocyanat) PDMI) und Mischungen daraus, 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, 2-Methyl-1,5-diisocyanatopentan, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerenge-mische, sowie cycloaliphatische Diisocyanate wie 1,4-, 1,3- oder 1,2-Diisocyanato-cyclohexan, 4,4'- oder 2,4'-Di(isocyanatocyclohexyl)methan, 1-Isocyanato-3,3,5-tri-methyl-5-(isocyanatomethyl)cyclohexan (Isophorondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyana-to-3,3,5-trimethyl-5-(isocyanatomethyl)cyclohexan und 4,4'-Di(isocyanatocyclohexyl)-methan. Besonders bevorzugt sind 1,6-Diisocyanatohexan, 1,5-Diisocyanatopentan, 1-Isocyanato-3,3,5-trimethyl-5-(isocyanatomethyl)cyclohexan und 4,4'-Di(isocyanatocyclohexyl)methan.

Geeignete Amine, die bei dem erfindungsgemäßen Verfahren mit Gasphasen-Phosgenierung zur Reaktion zu den korrespondierenden Isocyanaten eingesetzt werden können, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-aminomethylcyclohexan (IPDA) und 4,4-Diaminodicyclohexylmethan. Bevorzugt verwendet wird 1,6-Diaminohexan (HDA).

Ebenfalls können für das erfindungsgemäße Verfahren mit Gasphasen-Phosgenierung aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA sowie 2,4'- und/oder 4,4'-MDA.

Besonders bevorzugt für die Gasphasen-Phosgenierung nach dem erfindungsgemäßen Verfahren ist das Amin ausgewählt aus der Gruppe bestehend aus 1,6-Diaminohexan, monomeres 2,4'-Methylen(diphenylamin), monomeres 4,4'-Methylen(diphenylamin), 2,4-Toluylendiamin, 2,6-Toluylendiamin, 1-Amino-3,3,5-trimethyl-5-Aminomethylcyclohexan und Mischungen davon.

Für das erfindungsgemäße Verfahren mit Flüssigphasen-Phosgenierung besonders geeignete Amine sind beliebige primäre Mono- und Polyamine wie z.B. Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4-Diaminotoluol, 2,6-Diaminotoluol, Gemische der beiden letztgenannten Isomeren, 2,2'-Diaminodiphenylmethan, 2,4'-Diaminodiphenylmethan, 4,4'-Diaminodiphenylmethan, Gemische der drei letztgenannten Isomeren, Alkyl-substituierte Diamine der Diphenylmethanreihe, wie beispielsweise 3,4'-Diamino-4-methyldiphenylmethan, Polyamingemische der Diphenylmethanreihe, wie sie in an sich bekannter Weise durch Anilin-Formaldehyd-Kondensationen erhalten werden, p-Xylendiamin, perhydriertes 2,4- und/oder 2,6-Diaminotoluol, 2,2'-, 2,4'- und/oder 4,4'-Diaminodicyclohexylmethan, 1-Amino-3,3,5-trimethyl-5-aminomethyl-cyclohexan (Isophorondiamin, abgekürzt IPDA), Lysin-ethylester, Lysin-aminoethylester, 2,4- und 2,6-Toluydendiamin und 1,6,11-Triamino-undecan.

In den Figuren 1a bis 1c sind schematisch drei Möglichkeiten für die Durchführung der Phosgenierung dargestellt, um das erfindungsgemäße Verfahren zu veranschaulichen. In Figur 1a ist die übliche Phosgenierung von Aminen zur Isocyanten, bei der Mischen der Edukte und Umsetzen des Reaktionsgemischs jeweils in einem Strang durchgeführt werden, zu sehen (nicht erfindungsgemäß). In Figur 1b ist eine Ausführungsform der erfindungsgemäßen Variante (i), bei der die Mischzone aus zwei parallel geschalteten und getrennt voneinander regelbaren Strängen aufgebaut ist, die jeweils eine Mischeinheit umfassen, dargestellt. In Figur 1c ist eine Ausführungsform der erfindungsgemäßen Variante (iii), bei der sowohl die Mischzone als auch die Reaktionszone aus zwei parallel geschalteten und getrennt voneinander regelbaren Strängen aufgebaut sind, die jeweils eine Mischeinheit und eine Reaktionseinheit umfassen, gezeigt. "A" bedeutet dabei Amin, gegebenenfalls gemischt mit Lösungsmittel "(+S)", "P" kennzeichnet Phosgen, gegebenenfalls mit Lösungsmittel gemischt "(+S)", "S" steht für Lösungsmittel und "I" für Isocyanat. P1 > P2 bedeutet, dass in der ersten Reaktionseinheit der Druck höher liegt als in der zweiten Reaktionseinheit.

Im Folgenden wird die Erfindung anhand von Beispielen erläutert, die aus thermodynamischen und kinetischen Daten berechnet wurden.

### Vergleichsbeispiel 1:

In einer Anlage zur Herstellung von Toluylendiisocyanat (TDI) durch Phosgenierung von TDA in der Flüssigphase werden eine 30%ige Lösung von Toluylendiamin (TDA) in Monochlorbenzol mit einem Phosgenstrom in einer Mischdüse (Mischzeit 11,7 ms) vermischt. Das molare Verhältnis von Phosgen zu TDA beträgt 10. Die Mischtemperatur der Ströme beträgt etwa 60°C. Stromab der Mischdüse ist ein adiabater Rohrreaktor mit einer Verweilzeit von etwa 2 min installiert. In diesem erfolgt die Umsetzung des Amins zu Isocyanat bzw. zum Carbamylchlorid als Vorstufe. Durch adiabate Temperaturerhöhung sowie durch Freisetzung von HCl wird eine Gasphase ausgebildet. Das zweiphasige Reaktionsgemisch wird anschließend einer Reaktionskolonne zugeführt.

Die Ausbeute der Reaktionsstufe bezüglich TDA beträgt 93,4 %.

### Vergleichsbeispiel 2 :

Die oben beschriebene Anlage soll nur 50% ihrer Nennlast produzieren. Entsprechend werden aminhaltiger und phosgenhalter Eduktstrom halbiert. In deren Folge steigt die Mischzeit auf 23,4 ms. Gleichzeitig steigt die Verweilzeit im Reaktor auf das Doppelte. Die Ausbeute an TDI fällt von 93,4% auf 80,2%

### Erfindungsgemäßes Beispiel 1 :

In der oben beschriebenen Anlage werden erfindungsgemäß zwei parallele Düsen installiert. Jede davon ist für 50% der Gesamtnennlast der Anlage so ausgelegt, dass die in Beispiel 1 genannte Mischzeit realisiert wird. Im Betrieb der Anlage bei 50% der Nennlast wird eine der Düse abgesperrt. Die zweite Düse wird entsprechend ihrer Auslegung betrieben, so dass die Mischzeit bei 11,7 ms verbleibt. Entsprechend werden 93,4% Ausbeute bei gleichen spezifischen Energiekosten und damit ohne die Nachteile des Vergleichsbeispiels 2 erreicht.

## Patentansprüche

1. Verfahren zur Herstellung von Isocyanaten durch Umsetzung der korrespondierenden Amine mit Phosgen umfassend die Schritte
(a) Bereitstellen mindestens eines Amin enthaltenden Eduktstroms und mindestens eines Phosgen enthaltenden Eduktstroms,
(b) Mischen der Eduktströme zu mindestens einem Reaktionsgemisch in einer Mischzone,
(c) Umsetzen des mindestens einen Reaktionsgemischs in einer Reaktionszone, und
(d) Aufarbeiten des mindestens einen aus (c) erhaltenen Produktgemischs,
wobei
(i) die Mischzone aus mindestens zwei parallel geschalteten und getrennt voneinander regelbaren Strängen jeweils umfassend mindestens eine Mischeinheit aufgebaut ist,
wobei getrennt voneinander regelbar bedeutet, dass die einzelnen, parallel geschalteten Stränge voneinander separat absperrbar sind und jeweils unabhängig voneinander betrieben werden können.

2. Verfahren nach Anspruch 1, **dadurch gekennzeichnet, dass** in der(den) Mischeinheit(en) statische Mischorgane eingesetzt werden.

3. Verfahren nach Anspruch 1 oder 2, **dadurch gekennzeichnet, dass** die Reaktion in einer gemeinsamen Reaktionszone aufgebaut aus mindestens einer Reaktionseinheit erfolgt.

4. Verfahren nach Anspruch 3, **dadurch gekennzeichnet, dass** die Reaktionseinheit(en) mindestens einen Verweilzeitreaktor umfasst(en).

5. Verfahren nach Anspruch 4, **dadurch gekennzeichnet, dass** als Verweilzeitreaktoren Rohrreaktoren und/oder Rührkesselkaskaden eingesetzt werden.

6. Verfahren nach einem der Ansprüche 1 bis 5, **dadurch gekennzeichnet, dass** die Aufarbeitung in Schritt (d) in einem gemeinsamen Strang erfolgt.

7. Verfahren nach einem der Ansprüche 1 bis 6, **dadurch gekennzeichnet, dass** die Umsetzung in flüssiger Phase erfolgt.

8. Verfahren nach Anspruch 7, **dadurch gekennzeichnet, dass** in dem mindestens einen Amin enthaltenden Eduktstrom das Amin als Lösung oder als Suspension vorliegt.

9. Verfahren nach Anspruch 7 oder 8, **dadurch gekennzeichnet, dass** das Amin in einer Konzentration von 5 bis 95 Gew.-%, bezogen auf den bereitgestellten mindestens einen Amin enthaltenden Eduktstrom, eingesetzt wird.

10. Verfahren nach einem der Ansprüche 7 bis 9, **dadurch gekennzeichnet, dass** das Molverhältnis von Phosgen zu primärer Aminogruppe 15:1 bis 1:1 beträgt.

11. Verfahren nach einem der Ansprüche 7 bis 10, **dadurch gekennzeichnet, dass** das Amin ausgewählt ist aus der Gruppe bestehend aus Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4- und/oder 2,6-Diaminotoluol, 2,2'-, 2,4'- und/oder 4,4'-Diaminodiphenylmethan, Alkyl-substituierte Diamine der Diphenylmethanreihe, wie beispielsweise 3,4'-Diamino-4-methyldiphenylmethan, Polyamingemische der Diphenylmethanreihe, wie sie in an sich bekannter Weise durch Anilin-Formaldehyd-Kondensationen erhalten werden, p-Xylendiamin, perhydriertes 2,4- und/oder 2,6-Diaminotoluol, 2,2'-, 2,4'- und/oder 4,4'-Diaminodicyclohexylmethan, 1-Amino-3,3,5-trimethyl-5-aminomethylcyclohexan (Isophorondiamin, abgekürzt IPDA), Lysinethylester, Lysinaminoethylester, 2,4- und/oder 2,6-Toluydendiamin, 1,6,11-Triaminoundecan und Mischungen davon.

12. Verfahren nach einem der Ansprüche 7 bis 11, **dadurch gekennzeichnet, dass** als Lösungsmittel für die Edukte Monochlorbenzol, o-Dichlorbenzol, Trichlorbenzol, Toluol, Xylol, Methylenchlorid, Perchlorethylen, Trichlorfluoromethan, Butylacetat, Hexan, Heptan, Octan, Biphenyl, Essigsäureethylester, 1,2-Diacetoxyethan, 2-Butanon, Acetonitril, Sulfan oder Mischungen davon eingesetzt wird.

13. Verfahren nach einem der Ansprüche 1 bis 6, **dadurch gekennzeichnet, dass** die Umsetzung in gasförmiger Phase erfolgt.

14. Verfahren nach Anspruch 13, **dadurch gekennzeichnet, dass** das mindestens eine aus Schritt (c) erhaltene Produktgemisch in mindestens einer Quenchzone abgekühlt wird.

15. Verfahren nach einem der Ansprüche 13 bis 14, **dadurch gekennzeichnet, dass** die Umsetzung in Gegenwart mindestens eines Inertmediums durchgeführt wird.

16. Verfahren nach einem der Ansprüche 13 bis 15, **dadurch gekennzeichnet, dass** das Amin ausgewählt ist aus der Gruppe bestehend aus 1,6-Diaminohexan, monomeres 2,4'- Methylen(diphenylamin), monomeres 4,4'-Methylen(diphenylamin), 2,4-Toluylendiamin, 2,6-Toluylendiamin, 1-Amino-3,3,5-trimethyl-5-aminomethylcyclohexan und Mischung davon.
